# EUROPEAN PATENT APPLICATION

(11) **EP 3 489 902 A1**
(43) Date of publication of application: **29.05.2019**
(21) Application number: 17203542.0
(22) Date of filing: 24.11.2017
(51) Int. Cl.: G06T 11/00

(54) **METHOD FOR CREATING A PSEUDO CT IMAGE AND COMPUTER PROGRAM PRODUCT**

(71) Applicant: Koninklijke Philips N.V., 5656 AE Eindhoven (NL)
(72) Inventor: VAHALA, Erkki Tapani, 5656 AE Eindhoven (NL); KRISHNAIYER RAMAN, Sivaramakrishnan, 5656 AE Eindhoven (NL)
(74) Representative: de Haan, Poul Erik

(57) **Abstract**

It is an object of the invention to simplify MRI based radiotherapy workflow. This object is achieved by a method according to a method for creating a pseudo CT image comprising a region of interest. The pseudo CT image is created from one or more magnetic resonance images. The region of interest has been positioned on a tabletop of the magnetic resonance imaging system during the acquisition of the one or more magnetic resonance images. The method comprises the following steps:
receiving a value for a tabletop height, wherein the value for the tabletop height is related to the height of the tabletop of the magnetic resonance imaging system when the magnetic resonance system is in use and;
receiving magnetic resonance image data comprising one or more magnetic resonance images, wherein the one or more magnetic resonance images comprise the region of interest and are acquired by the magnetic resonance imaging system and;
creating a pseudo CT image by determining a pseudo Hounsfield Unit value for one or more voxels in the region of interest based on the one or more magnetic resonance images wherein the pseudo CT data comprise the value for the tabletop height;

## Description

### FIELD OF THE INVENTION

The invention relates to the field of radiotherapy and more specifically to a magnetic resonance based workflow for radiotherapy

### BACKGROUND OF THE INVENTION

In external beam radiation therapy planning, a treatment is simulated in the actual treatment position on a computed tomography (CT) or magnetic resonance imaging (MRI) scanner. Depending on the imaging modality used, CT or MRI images are acquired while the patient is in the treatment position. These acquired images are used in order to create a treatment plan. Treatment plan creation is typically done by means of a treatment planning system (TPS). In order to create a treatment plan, information is needed about the position of a treatment target (e.g. a tumor). Also the position of organs at risk need to be known. A treatment goal could be to apply a high radiation dose to the treatment target, while limiting the dose to the organs at risk. In addition to the positions of the treatment target and the organs at risk, in order to create a treatment plan, the treatment planning system needs to be provided with information on how radiation is attenuated in the body of a patient to be treated. This information can be derived directly from CT images. In general, CT images display Hounsfield units (HU). HU are a measure for radiation attenuation. However, this information is not directly available from MRI images. Methods exists that allow the derivation of a pseudo CT image from one or more MRI images. A pseudo CT image comprises pseudo Hounsfield Units, which are a measure for radiation attenuation. A workflow for creating a treatment plan based on MRI images, is herein called an "MRI based radiotherapy workflow".

In the MRI based radiotherapy workflow, the MRI tabletop is not visible in the pseudo CT image when the pseudo CT image is used for planning on the TPS. The presence of the tabletop is very relevant as it affects the radiation beams used for treatment. Therefore, a model of the tabletop used during treatment delivery is often used to create a representation of the treatment tabletop in planning images used for treatment planning. An operator of the system makes use of either the intensity gradient at the junction of skin and air of the image, or the patient surface contour as reference for positioning the representation of the treatment tabletop in the planning image. In some cases, a supporting material is used for the patient (such as a vacuum cushion) for immobilizing the patient during scan. In these cases, the operator is not being able to deduce the distance of the couch tabletop from the patient skin.

WO 2015/150065 A1 describes a method of creating a pseudo CT image. The method comprising the steps of: determination of a relative prevalence of a first tissue class and second tissue class within the volume element from a first magnetic resonance image and second magnetic resonance image respectively. Then a relative prevalence of a third tissue class is determined within the volume element based on subtraction of a relative prevalence of the first and/or second tissue class from a total tissue prevalence. A reference Hounsfield Unit value is provided for the first, second and third tissue class. Finally, a pseudo Housfield value is estimated for the volume element by determining a weighted sum of the first, second and third reference Hounsfield unit value, with weight factors which are based on the determined relative prevalences of the first, second and third tissue class.

### SUMMARY OF THE INVENTION

It is an object of the invention to simplify MRI based radiotherapy workflow. This object is achieved by a method according to claim 1 and a computer program product according to claim 5.

It is an insight of the inventors that the current approach with respect to incorporating a treatment table into the radiotherapy planning is rather complicated. In order to address this, the CT-based tabletop positioning method could be replicated with MRI. However, this approach requires the use of markers on the tabletop. When using this in MRI it would easily introduce aliasing and smearing artefacts to the clinical data and interfere with automatic contouring algorithms. Addition of markers with microcoils or with multinuclei receiver coils could remove the artifacts, but would also make the system more complicated and expensive.

It is a further insight of the inventors that the existing, positioning of the tabletop on pseudo CT images does not work on all patient groups. This method makes use of skin contrours. For some patients groups, vacuum cushions or other supporting materials are used to lift the patient off from the table during MR simulation and during the treatment itself, making the skin-surface an invalid positioning target.

Receiving a value for a table top height, wherein the value for the tabletop height is related to the height of the tabletop of the magnetic resonance imaging system when the magnetic resonance system is in use allows creation of a representation of a tabletop in the pseudo CT image based on this value for the tabletop height. In this way, the workflow may be simplified. Creation of the representation of the table top is not necessarily created by the same product or service as the product or service that creates the pseudo CT image. The creation of the pseudo CT image could for example be done on the magnetic resonance imaging system or by a separate computer program product or cloud based service. The representation of the tabletop could in turn be created by the treatment planning system.

The tabletop height is a measure for the distance between an isocenter of the MRI system and the tabletop. This could for example be the outside of the tabletop, but it could also be another (known) point in or on the tabletop, like e.g. its center of gravity. Instead of the isocenter also a different position can be taken to determine the distance from, as long as the resulting value for the tabletop height represents a value that can be used for the absolute positioning of a representation of a tabletop in the image in the direction substantially perpendicular to the tabletop, when the tabletop is positioned in its normal orientation.

According to the method according to embodiments of the invention, the pseudo CT data comprise the value for the tabletop height. This information could for example be comprised by the display of the representation of the tabletop in the pseudo CT image. Also, this information could be comprised as an attribute to a pseudo CT DICOM file

Preferably, the method according to the invention is implemented in a computer program product. Preferably, the computer program product is comprised in a treatment planning system. Magnetic resonance image data comprising one or more magnetic resonance images is received. The one or more magnetic resonance images are used to create a pseudo CT image. The magnetic resonance image data could also comprise the value for the tabletop height. For example, the value for the tabletop height could be included as a DICOM attribute. This embodiment is advantageous, because in this way the one or more magnetic resonance images are directly linked to the tabletop height and this may reduce the chance of errors.

The value for the table top height could be measured, e.g. by means of a phantom. Alternatively, this value can be provided by the MRI system manufacturer.

The value for the tabletop height can only be used to determine the position of the tabletop in one direction (e.g. the dorsal - ventral relative to a patient in the MRI or CT image). Additionally, it may be favorable to standardize the alignment in the left-right direction (relative to a patient anatomy in the MRI image). This can be achieved by detecting the outline of the patient or at least part thereof in the one or more magnetic resonance images and / or in the created pseudo CT image and using the detected outline to determine the position of the representation of the tabletop in the pseudo CT image in the left-right direction, wherein left and right are defined by the left and right part of the patient's anatomy, or the direction substantially parallel to the table top.

According to other embodiments of the invention, the detected patient outline is used as a quality check for the value of the tabletop height.

According to another aspect, embodiments of present invention are a computer program product, wherein the computer program product comprises program code means for causing a computer to carry out the steps of any of the methods described above.

According to embodiments of the invention, the value for the tabletop height is configured or set in the computer program product. This is advantageous, because in this way this information does not need to be incorporated into the magnetic resonance imaging data and therefore no or limited adaptations are required during the MRI acquisition process.

According to further embodiments of the invention, multiple values for the tabletop height are configured and linked to different magnetic resonance imaging systems. The computer program product selects the correct tabletop height for use upon identification of the magnetic resonance imaging system used for the acquisition of the one or more magnetic resonance images. The information for identifying the MRI system could for example be present in the (standard) DICOM headers or attributes.

These and other aspects of the invention will be apparent from and elucidated with reference to the embodiments described hereinafter.

### BRIEF DESCRIPTION OF THE DRAWINGS

Fig. 1 diagrammatically shows and MRI based radiotherapy workflow and
Fig. 2 diagrammatically shows a method according to embodiments of the invention.
Fig. 3 shows a pseudo CT image.

### DETAILED DESCRIPTION OF THE INVENTION

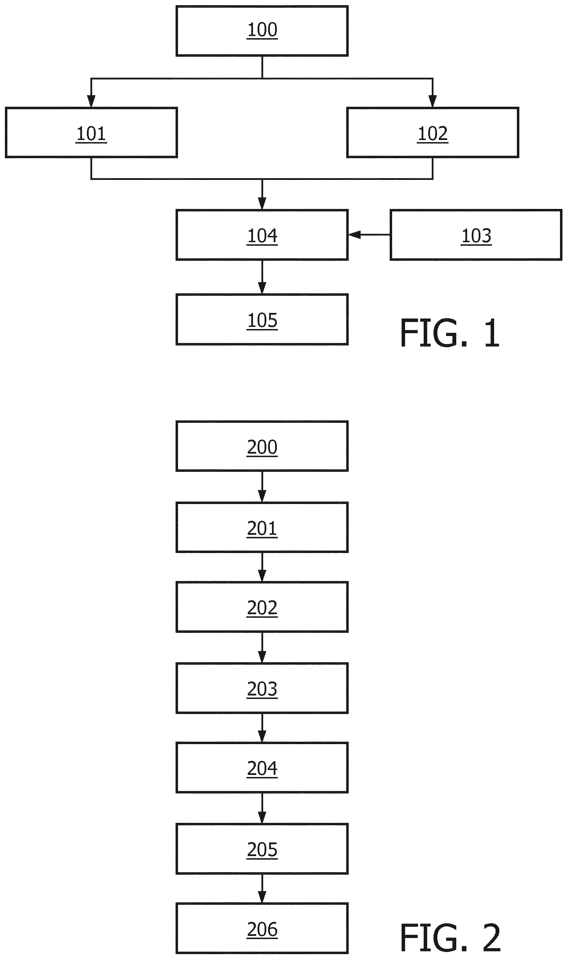

Fig. 1 diagrammatically shows and MRI based radiotherapy workflow. In step 100 MRI images from a region of interest (e.g. (part of) a patient or animal) are being acquired. These images are to be used for the creation of a pseudo CT image and for the planning of the radiotherapy delivery. These images could for example be DIXON images, UTE images, or separate T1 and T2 weighted image series. During the acquisition of the one or more magnetic resonance images, the region of interest is positioned on a tabletop of the MRI system. This tabletop is often invisible on the one or more MRI images. In step 101 from one or more magnetic resonance images a pseudo CT image is being created. Methods to create a pseudo CT image are known in the art. Such methods could for example be based on the voxel signal value in one or more of the MRI images. Also, creation of pseudo CT images could be based on segmentation of different tissue types in the one or more magnetic resonance images, or deep learning algorithms that classify or convert the voxels to HU values based on input voxel values. Also, creation of pseudo CT images could be a combination of these methods. The magnetic resonance images are also used for the delineation of a treatment target (e.g. tumor) and surrounding organs at risk (step 102). Often safety margins are added to the delineations in order to account for different sources of uncertainty (e.g. caused by motion or inaccuracies in the setup) Both the pseudo CT image and the delineation are an input for the treatment planning performed in step 104. In addition to that the treatment planning requires treatment objectives as an input, e.g. minimum and / or maximum dose per volume for the different delineated structures 103. In step 104 all these inputs are being used to create a treatment plan and in step 105 a radiation treatment is delivered according to the plan. Also during radiation treatment delivery the region of interest is positioned on a tabletop. In this case a treatment tabletop. The treatment tabletop does not necessarily have the same characteristics as the tabletop used for acquiring the planning / simulation magnetic resonance images.

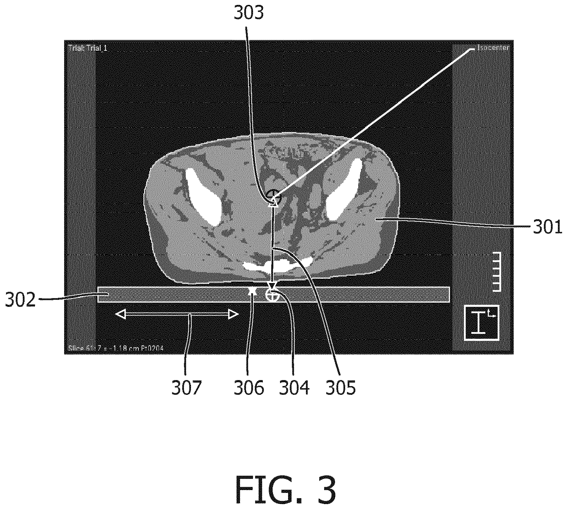

Fig. 2 diagrammatically shows a method according to embodiments of the invention. The method comprises the following steps:
receiving a value for a tabletop height, representing a height of the tabletop of the magnetic resonance imaging system 200. The value for the tabletop height can be for example determined based on measurements. This could be done by using a phantom with known geometrical dimensions. The phantom is then placed on the tabletop of the MRI system used to acquire magnetic resonance images for treatment planning / simulation purposes. A magnetic resonance image can then be acquired from the phantom such that the origin of the image matches with the isocenter of the MRI system Fig. 3, 303. The distance between the coordinate of the lowest part of the phantom and the isocenter can be used as a value of the tabletop height. When preferred, the (known) distance from the outside of the tabletop to its center of gravity can be added to the determined distance. In that way, the distance between the isocenter and the center of gravity of the tabletop is the value for tabletop height. The determination of the value for the tabletop height could be performed as part of routinely quality assurance measurements at the hospital. Alternatively, the value for the tabletop height could be provided by the manufacturer of the MRI system. The value of the tabletop height could be received as part of the magnetic resonance imaging data, for example this information could be comprised in a private DICOM attribute.
receiving magnetic resonance image data comprising one or more magnetic resonance images, wherein the one or more magnetic resonance images comprise the region of interest and are acquired by the magnetic resonance imaging system 201 and;
creating a pseudo CT image by determining a pseudo Hounsfield Unit value for one or more voxels in the region of interest based on the one or more magnetic resonance images 202 and;
creating a representation of a tabletop in the pseudo CT image based on the value for the tabletop height 203. It is important to realize that the tabletop used for planning / simulation has not necessarily the same characteristics as the tabletop used during treatment delivery. In addition to that different treatments systems, like e.g. linear accelerator (LINAC) may comprise tabletops having different characteristics. A tabletop model associated with the LINAC used in treatment can be loaded to be used for the treatment plan calculations. This tabletop model can be positioned using the distance as an offset in the direction (substantially) perpendicular to the tabletop. Most often, this is substantially the same direction as the ventral-dorsal direction of the patient in the image.

Optionally, an outline of the patient in the one or more magnetic resonance images and / or in the created pseudo CT image is detected 204. This could for example be achieved by means of a histogram to detect background noise level. Thresholding the image with a heuristic, fractional threshold noise-level value gives a body outline mask, in general regardless of the absolute pixel values.

The detected outline could be used to determine the position of the representation of the tabletop in the pseudo CT image in the left-right direction, wherein left and right are defined by the left and right part of the patient's anatomy 205, or the direction substantially parallel to the tabletop Fig. 3, 307.

Additionally or alternatively, the detected outline could be used as a quality check for the value of the tabletop height. For example if the representation of the tabletop will be positioned within the detected outline based on the value for the tabletop height, this could be a reason for alerting the operator to check if an error occurred. Also, when the representation of the tabletop will be positioned too far from the detected outline this may be a reason to alert the operator.

Fig. 3 shows a pseudo CT image 301 of a patient. In the image the patient is positioned on a representation of a tabletop (e.g. tabletop model) 302. 303 shows the isocenter. The value for the tabletop height could for example be given by the (shortest) distance 305 between the isocenter 303 and an outline of the representation of the tabletop 302. As an alternative the distance of between the outline of the tabletop and the center of gravity of the tabletop 306 may be added to 305. These values combined could also serve as the value for the tabletop height.

Whilst the invention has been illustrated and described in detail in the drawings and foregoing description, such illustrations and description are to be considered illustrative or exemplary and not restrictive; the invention is not limited to the disclosed embodiments.

## Claims

1. A method for creating a pseudo CT image comprising a region of interest, wherein the pseudo CT image is created from one or more magnetic resonance images acquired by means of a magnetic resonance imaging system, wherein the region of interest has been positioned on a tabletop of the magnetic resonance imaging system during the acquisition of the one or more magnetic resonance images, wherein the method comprises the following steps:
receiving a value for a tabletop height, wherein the value for the tabletop height is related to the height of the tabletop of the magnetic resonance imaging system when the magnetic resonance system is in use and;
receiving magnetic resonance image data comprising one or more magnetic resonance images, wherein the one or more magnetic resonance images comprise the region of interest and are acquired by the magnetic resonance imaging system and;
creating a pseudo CT image by determining a pseudo Hounsfield Unit value for one or more voxels in the region of interest based on the one or more magnetic resonance images wherein the pseudo CT data comprise the value for the tabletop height;

2. A method for creating a pseudo CT image according to claim 1, further comprising the step of
creating a representation of a tabletop in the pseudo CT image using the value for the tabletop height.

3. A method for creating a pseudo CT image according to claim 1 or 2, wherein the magnetic resonance image data comprise the value for the tabletop height.

4. A method for creating a pseudo CT image according to any of claims 1-3 wherein the value for the tabletop height is measured or factory provided.

5. A method for creating a pseudo CT image according to any of claims 2-4, wherein the region of interest comprises an outline of a patient and wherein the method comprises the steps of:
detecting the outline of the patient in the one or more magnetic resonance images and / or in the created pseudo CT image and;
using the detected outline to determine the position of the representation of the tabletop in the pseudo CT image in the left-right direction, wherein left and right are defined by the left and right part of the patient's anatomy or as the direction substantially parallel to the tabletop in the pseudo CT image or the one or more magnetic resonance images.

6. A method for creating a pseudo CT image according to any of claims 2-5, wherein the region of interest comprises an outline of a patient and wherein the method comprises the steps of:
detecting the outline of the patient in the one or more magnetic resonance images and / or in the created pseudo CT image and;
using the detected outline as a quality check for the value of the tabletop height.

7. Computer program product configured for creating a pseudo CT image, wherein the computer program product comprises program code means for causing a computer to carry out the steps of a method of according to any of claims 1-6.

8. Computer program product according to claim 7, wherein the value for the tabletop height is configured in the computer program product.

9. Computer program product according to claim 8, wherein multiple values for the tabletop height are configured and linked to different magnetic resonance imaging systems and wherein the computer program product selects the correct tabletop height for use upon identification of the magnetic resonance imaging system used for the acquisition of the one or more magnetic resonance images.
